(19) Europäisches Patentamt / European Patent Office / Office européen des brevets

(11)  **EP 3 767 364 B1**

(12)  **EUROPEAN PATENT SPECIFICATION**

(45) Date of publication and mention
of the grant of the patent:
**24.04.2024  Bulletin 2024/17**

(21) Application number: **20160417.0**

(22) Date of filing: **02.03.2020**

(51) International Patent Classification (IPC):
**G02B 9/62** *(2006.01)*      **G02B 9/64** *(2006.01)*
**G02B 13/00** *(2006.01)*     **G02B 13/06** *(2006.01)*
**G02B 23/24** *(2006.01)*     **A61B 1/00** *(2006.01)*

(52) Cooperative Patent Classification (CPC):
**G02B 13/06; G02B 9/62; G02B 9/64;
G02B 13/0045; G02B 23/243;** A61B 1/00096

(54)  **WIDE FIELD OF VIEW ENDOSCOPE**

ENDOSKOP MIT BREITEM SICHTFELD

ENDOSCOPE À GRAND CHAMP DE VISION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.07.2019  EP 19187218**

(43) Date of publication of application:
**20.01.2021  Bulletin 2021/03**

(73) Proprietor: **Hoya Corporation**
**Tokyo 160-8347 (JP)**

(72) Inventors:
• **MAYER, Wolfgang**
**86316 Friedberg (DE)**
• **VIEBACH, Thomas**
**86316 Friedberg (DE)**
• **ZISCHKE, Holger**
**86316 Friedberg (DE)**
• **SCHROETER, Tilman**
**86316 Friedberg (DE)**

(74) Representative: **TBK**
**Bavariaring 4-6**
**80336 München (DE)**

(56) References cited:
**EP-A1- 1 629 764**       **WO-A1-2009/150653**
**WO-A1-2017/027749**    **JP-A- H10 309 259**
**JP-A- 2008 058 387**     **US-A1- 2007 213 590**
**US-A1- 2017 146 778**

**Description**

Field of the invention

The present invention is related to an endoscope with a wide field of view, such as more than 180°, and a rigid tip portion thereof. In particular, the endoscope and rigid tip portion, respectively, are suitable for being inserted into a lumen of a human body.

Background of the invention

A main target of today's endeavours in the field of endoscopy for medical purposes (in particular, in the field of diagnostics of the human body) is to increase the adenoma detection rate by the physicians. For this purpose, it is desired to increase the field of view (FoV) of endoscopes to more than 180°, preferably at least 200°. With such wide FoVs (WFoV), the physicians are able to observe the area behind a fold of the gastrointestinal tract or the respiratory tract or a blood vessel without complex manoeuvres.
Conventional endoscopes for inserting into a lumen (such as the gastrointestinal tract, the respiratory tract, or a blood vessel) of a human body have a maximum FoV of 170° only.
Further cited prior art documents are EP 1 629 764 A1 showing an endoscope and endoscope tip forming material, JP H10 309259 A showing an endoscope, WO 2017/027749 A1 showing a fully integrated, disposable tissue visualization device, US 2007/0213590 A1 showing an apparatus and methods for examining, visualizing, diagnosing, manipulating, treating and recording of abnormalities within interior regions of body cavities, US 2017/0146778 A1 showing a wide-angle lens and JP 2008 058387 A showing a superwide-angle lens and WO 2009/150653 A1 showing an optical system for use in an endoscope.

Summary of the invention

It is an object of the invention to improve the prior art. Namely, according to aspects of the invention, there are provided a rigid tip portion for an endoscope for inserting into a lumen of a human body and an endoscope according to the respective independent claims. Further details are set out in the dependent claims.
According to some embodiments of the invention, the physician using the endoscope has a larger field of view. Thus, he/she can observe easily the area behind a fold of the wall of a lumen (such as the gastrointestinal tract, the respiratory tract, or a blood vessel) of the human body into which the endoscope is inserted, without performing complex manoeuvres to position the rigid tip portion of the endoscope such that the physician can observe the area behind the fold, as required with a conventional endoscope.
Further advantages become apparent from the following detailed description.
It is to be understood that any of the above modifications and the examples described below can be applied singly or in combination to the respective aspects to which they refer, unless they are explicitly stated as excluding alternatives.

Brief description of the drawings

Further details, features, objects, and advantages are apparent from the following detailed description of preferred embodiments of the invention, which is to be taken in conjunction with the appended drawings, wherein:

Fig. 1 depicts a distal portion of an endoscope;
Fig. 2 depicts a cross-section of a rigid tip portion of an endoscope accommodating a sensor system according to some embodiments of the invention;
Fig. 3 depicts a plan view on the distal end of the rigid tip portion according to some embodiments of the invention;
Fig. 4 shows an endoscope with a very long rigid tip portion inserted into the intestine of a human body;
Fig. 5 shows an endoscope according to some embodiments of the invention inserted into the intestine of a human body;

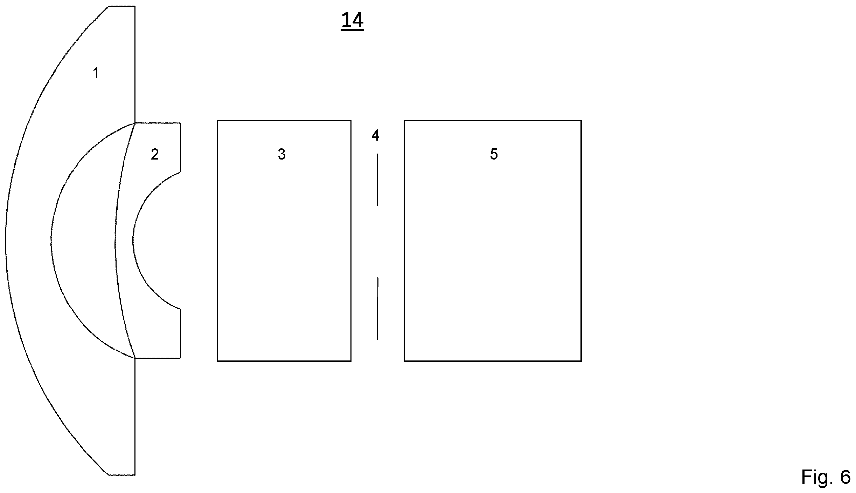

Fig. 6 depicts the general setup of an objective lens that may be used in a rigid tip portion according to some embodiments of the invention;
Fig. 7 shows the general setup of a sensor system that may be used in a rigid tip portion according to some embodiments of the invention;
Fig. 8 shows an embodiment 1 of a sensor system that may be used in a rigid tip portion according to the invention;
Fig. 9 shows an embodiment 2 of a sensor system that may be used in a rigid tip portion according to the invention;
Fig. 10 shows an embodiment 3 of a sensor system that may be used in a rigid tip portion according to the invention;

Fig. 11 shows an embodiment 4 of a sensor system that may be used in a rigid tip portion according to the invention;
Fig. 12 shows an embodiment 5 of a sensor system that may be used in a rigid tip portion according to the invention;
Fig. 13 shows an embodiment 6 of a sensor system that may be used in a rigid tip portion according to the invention;
Fig. 14 shows an embodiment 7 of a sensor system that may be used in a rigid tip portion according to the invention;
Fig. 15 shows an embodiment 8 of a sensor system that may be used in a rigid tip portion according to the invention; and
Fig. 16 shows a comparative example 9 of a sensor system that may be used in a rigid tip portion (not covered by the claimed invention).

Detailed description of certain embodiments

Hereinbelow, certain embodiments of the present invention are described in detail with reference to the accompanying drawings, wherein the features of the embodiments can be freely combined with each other unless otherwise described. However, it is to be expressly understood that the description of certain embodiments is given by way of example only, and that it is by no way intended to be understood as limiting the invention to the disclosed details.

Sometimes, the same reference signs are used throughout several figures. They designate the same or corresponding members.

In the field of endoscopy of the human body, it has to be ensured that the rigid tip portion of this WFoV endoscope is neither too long not too wide such that it can be used as a full functional therapeutic endoscope, e.g., in an Endoscopic submucosal dissection (ESD) procedure. Thus, a rigid tip portion 10 of an endoscope for inserting into a lumen (such as the gastrointestinal tract, the respiratory tract, or a blood vessel) of a human body must not be longer than 25 mm, preferably not longer than 20 mm, and still more preferably not longer than 15 mm. Its width (diameter of the cross section) must not be larger than 18 mm, preferably not larger than 16 mm, and still more preferably not larger than 14 mm.

The wall of the rigid tip portion 10 typically is a hollow cylinder with a circular cross-section, e.g. made of stainless steel or titanium. However, the rigid tip portion may have one or more indentations or bulges. The wall of the rigid tip portion 10 may be made of a single member or out of plural members which are rigidly connected to each other. Its cross-section may be even elliptical. In general, the rigid tip portion 10 has a center axis. At each position along the center axis, a respective cross-section of the rigid tip portion 10 perpendicular to the center axis is elliptical with a long axis and a short axis. The short axis is not longer than the long axis; in case of a circular cross-section, the short axis has a same length as the long axis. The long axis and the short axis cross on the center axis.

In order to allow insertion into a lumen of the human body, a maximum length of the long axis of the elliptical cross-section (i.e., a diameter of the circle in case of a circular cross-section) is preferably not more than 18 mm, more preferably not more than 16 mm, and still more preferably not more than 14 mm.

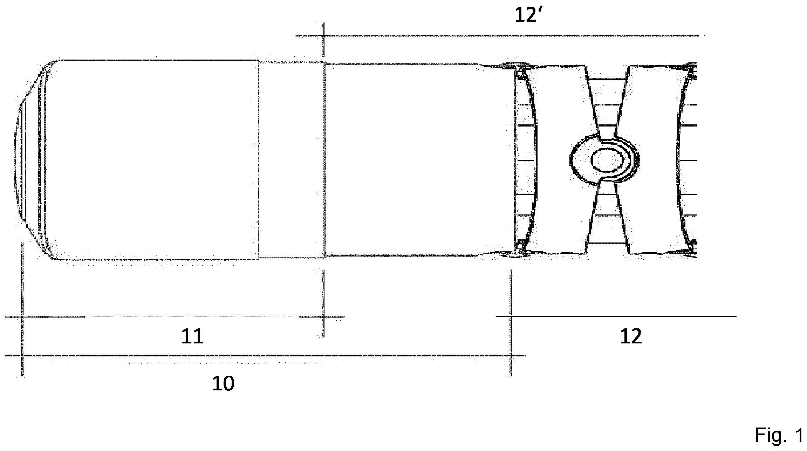

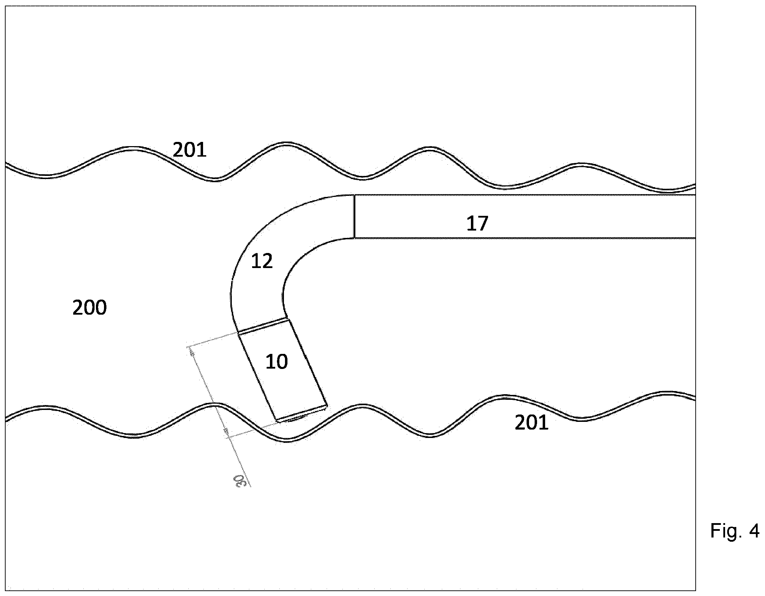

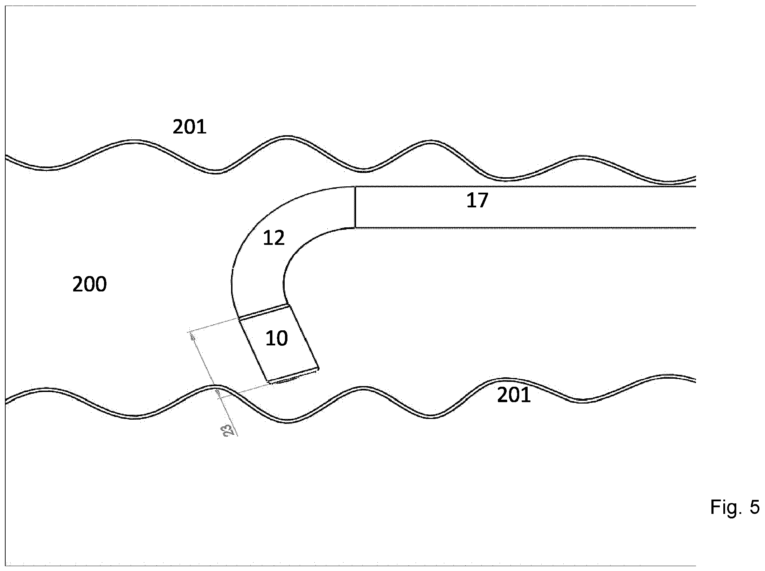

In some embodiments of the invention, the rigid tip portion is arranged at the distal end of an endoscope. It is typically connected to the proximal end by a angulation segment 12 and a flexible tube 17 (shown in Figs. 4 and 5). Sometimes, as shown in Fig. 1, it is said that the rigid tip portion 10 comprises a distal tip and the distal part of the angulation segment 12', which is the proximal part of the rigid tip portion 10. However, in the context of the present application, the term "angulation segment" is defined such that it does not comprise the proximal part of the rigid tip portion 10. That is, an endoscope comprises in a sequence from the distal end to the proximal end the rigid tip portion 10, the angulation segment 12 connected to the rigid tip portion 10, and the flexible tube 17 connected to the angulation segment 12.

The "angulation segment" 12 is the actively manoeuvrable portion of the endoscope. That is, the angulation segment is steerable in up to 4 directions (up/down, left/right). In contrast, the flexible tube 17 is flexible but not actively steerable.

In some embodiments of the invention, rigid tip portion 10 may be used stand-alone (i.e. without being connected to an angulation segment and a flexible tube) as a free-floating endoscope in so called "capsule endoscopy". In some embodiments of the invention, the rigid tip portion 10 may be connected directly with the flexible tube 17, without an angulation segment in between.

The rigid tip portion has sufficient rigidity not to be damaged or deformed when inserted into and moved through the lumen of the human body in a way as typically done by physicians.

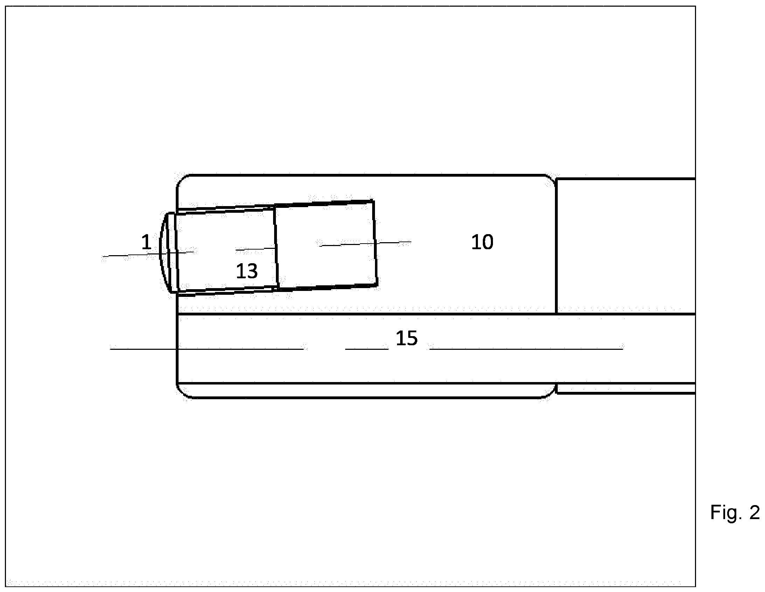

Fig. 2 shows a cross-section of the rigid tip portion 10. As can be seen in Fig. 2, the rigid tip portion 10 accommodates an objective lens 14 (a single objective lens) and an image sensor 7 to capture the image formed by the objective lens. The objective lens and the image sensor form a sensor system 13, and the sensor area (for photoelectrically converting an optical image taken through the single objective lens into an electrical image signal) of the sensor system is arranged in the image plane of the objective lens 14. The objective lens 14 may be fully accommodated within the rigid tip portion 10, or at least a part of a front lens 1 of the objective lens 14 may protrude from the distal end of the rigid tip portion 10.

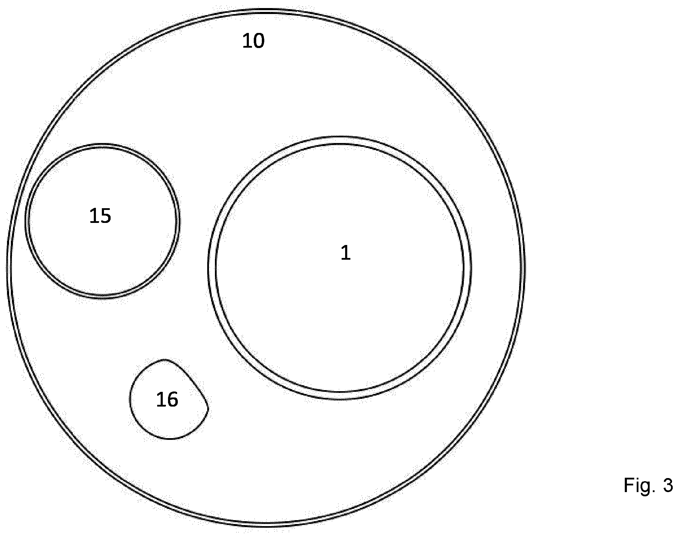

In addition, the rigid tip portion typically comprises an illumination unit in order to illuminate the scene captured by the objective lens. Furthermore, the rigid tip portion may comprise some circuits for controlling the image sensor and/or the illumination unit and/or to process the image signal from the image sensor. The rigid tip portion may also comprise other components such as a working channel 15 allowing the physician to perform some treatment in the lumen or on the wall of the lumen of the human body and an air/water nozzle 16 for cleaning the front surface of the front lens of the objective lens 14. Some of this members are seen in the plan view on the distal end of the rigid tip portion shown in Fig. 3.

The front lens 1 and the other members, if provided, are embedded in a front plate closing the rigid tip portion 10 at its distal end. The proximal end may be closed or open. The working channel 15, if any, electrical wirings, and optical fibers, if any, pass through the proximal end of the rigid tip portion 10 towards the proximal end of the endoscope, unless the rigid tip portion 10 is used as a free-floating endoscope. In this case, the proximal end is closed, too.

Typically, an optical axis of the objective lens 14 is arranged coincident with the central axis of the rigid tip portion 10. However this arrangement is not mandatory. The optical axis may be shifted relative to the central axis in order to accommodate some component (e.g. working channel 15) in the resulting space. Also, the optical axis may be tilted relative to the central axis, as shown in the example of Fig. 2. Thus, the field of view in one direction is enlarged at the cost of a reduced field of view in the opposite direction. The maximum tilt angle may be 15°, i.e., the image plane of the objective lens and the central axis of the rigid tip portion form an angel of not less than 75°.

In order to allow the physician to manoeuvre within the lumen of the human body, a length of the rigid tip portion should preferably not exceed 25 mm, more preferably not exceed 22 mm, and still more preferably not exceed 19 mm. This can be seen in Figs. 4 and 5, which show an endoscope inserted into an intestine 200 having intestinal walls 201. Fig. 4 shows an endoscope with a rigid tip portion having a length of 30 mm, while Fig. 5 shows an endoscope with a rigid tip portion having a length of 23 mm. From a comparison of Figs. 4 and 5, one sees that manoeuvring with the endoscope of Fig. 4 is much more difficult than manoeuvring with the endoscope of Fig. 5. That is, the length of the rigid tip portion should not exceed 30 mm, and preferably it is not more than 25 mm, more preferably not more than 23 mm, and still more preferably not more than 20 mm.

Furthermore, at least the front lens 1 of the objective lens 14 in the rigid tip portion 10 of an endoscope for inserting into a lumen of a human body should have a minimum thickness in order to ensure that it does not break during operation. In particular, it should be ensured that the lenses of the objective lens 14 do not break within the lumen of the human body. Therefore, the front lens 1 has a thickness of not less than 0.45 mm at its center and not less than 0.35 mm at its edge, where the front lens 1 is fixed by its lens holder (in other words: the thickness at the edge of the front lens 1 is measured at the inner edge of the lens holder of the front lens). More preferably, these values are 0.50 mm and 0.45 mm, respectively. It is preferred that the front lens 1 is made of glass because glasses are typically inert with respect to the substances present inside the human body (such as digestive enzyms, stool, urine, blood, etc.). However, the front lens 1 may be made of an appropriate plastic instead.

Furthermore, it is generally preferred that radii of curvature of the lenses in the single objective lens 14 are not too small in order to allow high precision manufacturing. Preferably, a minimum radius of curvature is 0.60 mm, more preferably 0.66 mm, and still more preferably 0.72 mm.

Some embodiments of the invention provide a rigid tip portion 10 of an endoscope for inserting into a lumen of a human body which fulfils these requirements and comprises a single objective lens 14 having a field of view (FoV) of not less than 180°, preferably not less than 200°. Preferably, the FoV is not less than 220° or even not less than 230°.

The rigid tip portion 10 also comprises an image sensor. The sensor area 7 (imaging surface) of the image sensor is arranged in an image plane of the single objective lens 14 (within some tolerances such as manufacturing tolerances, e.g. ± 1 mm, or ± 0.5 mm, or ± 0.2 mm). Typically, the image plane is perpendicular to an optical axis of the single objective lens 14 (i.e., the single objective lens 14 typically has rotational symmetry, but this is not mandatory) such that the image sensor is typically arranged in a plane perpendicular to the optical axis (within some tolerances such as manufacturing tolerances, e.g. ± 5°, or ± 2°, or ± 1°).

The image plane is located such that the objective lens 14 images a portion of a sphere or of an ellipsoid on the image plane. The sphere has a radius of not less than 8 mm and not more than 12 mm. A center of the sphere is located not less than 0 mm and not more than 4 mm towards the image sensor from an apex of the objective lens on the object side (i.e., a side opposite to a side where the image sensor is located). The depth of field may range from a minimum radius of 0 mm (rather: from the front surface of the front lens) or a sphere around the center having a minimum distance from the front surface of the lens of not more than 2 mm, to a maximum radius of 50 mm, preferably 75 mm, still more preferably 100 mm around the center. The depth of field (DOF) defines the distance between the nearest and the farthest objects that are in acceptably sharp focus in the image plane. It may be expressed as $DOF = 2\,u^2NC/f^2$ for a given circle of confusion c, focal length f, F-number N, and distance to the subject u. Here, the circle of confusion is assumed to have a diameter of not more than 7 $\mu$m, preferably not more than 6 $\mu$m, and even more preferred of not more than 4.05 $\mu$m.

The single objective lens 14 may comprise plural lens units. They are arranged along the optical axis of the single objective lens (or generally: arranged along a line perpendicular to the sensor area of the image sensor). Each lens unit may comprise one or more lenses. Some of the lens units may comprise cemented lenses.

The image sensor comprises a two-dimensional array of pixels arranged in the sensor area 7. Each of the pixels is configured to photoelectrically convert light incident on the pixel into a respective electrical pixel signal. For example, the sensor may be a CCD sensor or a CMOS sensor. The rigid tip portion 10 may comprise a part of the control circuit and/or processing circuit of the image sensor. Each of these circuits may be arranged fully or partly outside the endoscope, connected to the proximal end of the endoscope. For example, the image sensor may comprise a matrix of not less than 1024 pixels or 2048 pixels or 4096 pixels arranged in the image plane. The electrical image signal used to generate an image on a display device (such as a screen or a printout) may comprise the electrical pixel signals of a plurality of the pixels (e.g. of all the pixels). A pixel pitch may be e.g. 0.9 $\mu$m to 1.4 $\mu$m, and in particular 1.12 $\mu$m. Hereinafter, some objective lenses 14 used in different rigid tip portions 10 according to some embodiments of the invention are described at greater detail. However, the invention is not limited to these embodiments or to the principles disclosed hereinafter, as long as the respective rigid tip portion 10 comprising the objective lens 14 is suitable for inserting into a lumen of a human body.

Namely, the single objective lens 14 consists of, in order from an object side to an image side: two meniscus lenses (a first meniscus lens 1 being the front lens and a second meniscus lens 2), each having a convex side towards the object side; a first condensing unit 3 which condenses the light from the second meniscus lens 2; an aperture stop 4; a second condensing unit 5 which condenses the light from the aperture stop 4. Each of the first and second condensing units 3, 5 may comprise one or more lenses and the total number of lenses in the two condensing units 3, 5 is not less than four and not more than six. An apex of the second meniscus lens 2 is spaced apart from the lens surface on the image side of the first meniscus lens 1.

According to the invention and the embodiments of the invention (in particular embodiments 1 to 8 described below), the radius of the first meniscus lens 1 on the image side (r2), the radius of the second meniscus lens 2 on the object side (r3) and the radius of the second meniscus lens 2 on the image side (r4) fulfil at least two of the following three conditions:

$$r2/r4 \geq 1.40 \qquad (1);$$

$$r4/r3 \geq 0.111 \qquad (2);$$

and

$$r2/r3 \geq 0.16 \qquad (3).$$

The meaning of these conditions is as follows :

According to condition (1), the curvatures on the image side of the first and second meniscus lenses 1, 2 are substantially equal, or the curvature on the image side of the second meniscus lens 2 is stronger than that of the first meniscus lens 1. In the prior art, this quotient is typically smaller than 1, which means that the curvature of the image side of the first meniscus lens is stronger than that of the second meniscus lens. If condition (1) is not fulfilled, f/$\theta$-distortion is enhanced. In addition, the modulation transfer function at 150 lines per mm (MTF@150lp/mm) at a viewing angle of 115° decreases if condition (1) is not fulfilled (e.g. due to a decrease of r2 while r3 and r4 are maintained).

The second condition (2) defines that the curvature on the object side of the second meniscus lens 2 is much less than that on the image side of the second meniscus lens 2. However, it also defines that the curvature on the object side of the second meniscus lens 2 is not negligible compared to that on the image side.

In the prior art, the second lens of such an objective lens with a wide field of view (a fisheye objective) is substantially plano-convex, and in some cases, it may even be a biconcave lens. In contrast to the prior art, according to some embodiments of the invention, the second meniscus lens 2 is a "true" meniscus lens rather than substantially a plano-concave lens or a biconcave lens.

If condition (2) is not fulfilled, the MTF@150lp/mm at a viewing angle of 115° decreases (e.g. due to an increase of r3 while r2 and r4 are maintained). Also, the FoV may decrease.

The third condition (3) defines that the curvature on the image side of the first meniscus lens 1 is stronger than that on the object side of the second meniscus 2 lens. That is the reason why the apex of the second meniscus lens 2 is spaced apart from the first meniscus lens 1. Condition (3) also defines that the curvature on the object side of the second meniscus lens 2 is not negligible compared to the curvature on the image side of the first meniscus lens 1. As discussed with condition (2), in the prior art, the surface on the object side of the second lens is typically substantially flat (a plano-concave lens or even a biconcave lens). In some embodiments of the invention, the curvature on the object side of the second meniscus lens 2 must not be considered as substantially flat. Thus, the surface on the image side of the first meniscus lens 1 and the surface on the object side of the second meniscus lens 2 form a kind of a meniscus air lens.

If condition (3) is not fulfilled, the MTF@150lp/mm at a viewing angle of 115° decreases (e.g. due to a decrease of r2 while r3 and r4 are maintained, or due to a increase of r3 while r2 and r4 are maintained). Also, the FoV may decrease.

Preferably, at least one of the following conditions (1'), (2'), and (3') is fulfilled:

$$r2/r4 \geq 1.55 \qquad (1');$$

$$r4/r3 \geq 0.131 \qquad (2');$$

and

$$r2/r3 \geq 0.20 \qquad (3').$$

More preferably, at least one of the following conditions (1"), (2"), and (3") is fulfilled:

$$r2/r4 \geq 1.64 \qquad (1'');$$

$$r4/r3 \geq 0.152 \qquad (2'');$$

and

$$r2/r3 \geq 0.24 \qquad (3'').$$

Still more preferably, at least one of the following conditions (1'''), (2'''), and (3''') is fulfilled:

$$r2/r4 \geq 1.64 \qquad (1''');$$

$$r4/r3 \geq 0.19 \qquad (2''');$$

and

$$r2/r3 \geq 0.35 \qquad (3''').$$

Preferably, at least one of the following conditions is additionally fulfilled:

$$1.98 \geq r2/r4 \qquad (4);$$

$$0.35 \geq r4/r3 \qquad (5);$$

and

$$0.65 \geq r2/r3 \qquad (6).$$

If at least one of conditions (4) to (6) is fulfilled, it is ensured that the respective lens may be easily manufactured. That is, in these cases, it is not required that the respective surface is a half-sphere. Furthermore, the MTF@150lp/mm at a viewing angle of 115° decreases if conditions (5) and (6) are not fulfilled (e.g. due to a larger r3 at constant r4 and r2). Also, if conditions (4) and (6) are not fulfilled (e.g. due to a larger r2 at constant r4 and r3), the MTF@150lp/mm at a viewing angle of 115° decreases and the FoV decreases due to vignetting.

More preferably, at least one of the following conditions is additionally fulfilled:

$$1.94 \geq r2/r4 \qquad (4');$$

$$0.32 \geq r4/r3 \qquad (5');$$

and

$$0.61 \geq r2/r3 \qquad (6').$$

Still more preferably, at least one of the following conditions is additionally fulfilled:

$$r2/r4 \geq 1.90 \qquad (1'''');$$

$$0.24 \geq r4/r3 \qquad (5'');$$

and

$$0.40 \geq r2/r3 \qquad (6'').$$

According to some embodiments of the invention, the objective lens has a field of view of not less than 225°. Preferably, the field of view is not less than 230°, or even not less than 234°. On the other hand, in order to facilitate manufacturing of the objective lens, the field of view may be not more than 240°, preferably not more than 236°.

Note that the field of view is defined such that the image circle of a sphere having a distance of 8 mm from the apex of the first meniscus lens has a diameter of 2.08 mm to 2.18 mm, and a chief ray angle is not larger than 28° at each position in the image circle. Thus, a conventional semiconductor image detector, such as Omnivision OV5670, may be used to capture the image without vignetting.

According to some embodiments of the invention, if a radius of the first meniscus lens 1 on the object side is denoted r1, the following condition (7) is fulfilled:

$$4.0 \leq r1/r4 \leq 7.6 \qquad (7).$$

More preferably, one of the following conditions (7') and (7") is fulfilled:

$$4.0 \leq r1/r4 \leq 6.0 \qquad (7');$$

$$4.5 \leq r1/r4 \leq 5.5 \qquad (7'').$$

The first meniscus lens has a largest diameter among all the lenses comprised in the objective lens 14. A diameter of the first meniscus lens 1 may not be larger than the diameter of the rigid tip portion. However, in particular if the rigid tip portion 10 comprises other components at its distal end, too (such as a working channel 15, an air/water nozzle 16, and/or an illumination unit, see e.g. Fig. 3), the diameter of the first meniscus lens 1 is preferably not larger than 7 mm.

According to some embodiments of the invention, the sum of the refractive indices of the first and second meniscus lenses 1, 2 is not less than 3.56, and/or a sum of the Abbe-numbers of the first and second meniscus lenses is not less than 58. The refractive indices are determined at a wavelength of 587.6 nm. The Abbe number is defined as

$vd = (n_d-1)/(n_F - n_C)$, wherein $n_d$, $n_F$ and $n_C$ are the refractive indices of the material at the wavelengths of the Fraunhofer d-, F- and C- spectral lines (587.6 nm, 486.1 nm, and 656.3 nm, respectively). Preferably, the sum of the refractive indices is not less than 3.8 or even not less than 4. Correspondingly, the sum of the Abbe-numbers is preferably not less than 60 and even more preferable not less than 70.

According to some embodiments, the first condensing unit 3 consists of one, two, or three lenses. For example, the first condensing unit 3 may consist of a single biconvex lens. As another example, the first condensing unit 3 may consist of a biconvex lens and a meniscus lens having a concave side towards the object side. These lenses may be cemented. As a still further option, the first condensing unit 3 may consist of a meniscus lens having a concave side towards the object side and a plano-convex lens. As an example with three lenses, the first condensing unit 3 may consist of a biconcave lens, and two plano-convex lenses, wherein the biconcave lens and the first of two plano-convex lenses may be cemented. As another option, the first condensing unit 3 may consist of a plano-concave lens and a plano-convex lens, wherein the two planar surfaces may be cemented, and a plano-convex lens. These examples are not limiting.

Correspondingly, the second condensing unit 5 may consist of two, three or four lenses. For example, the second condensing unit 5 may consist of a single unit (cemented lenses) comprising two or three lenses, which may be cemented. This unit may also be useful to correct chromatic aberration and to ensure that the chief ray angle is not larger than a predetermined value such as 30° or preferably 28°. In addition, the second condensing unit 5 may comprise a further unit consisting of a single lens or a doublet, wherein the further unit is closer to the aperture stop than the former unit. These examples are not limiting.

According to some embodiments of the invention, the objective lens has small distortions. For example, the modulation transfer function (MTF) at 300 line pairs per mm (MTF@300lp/mm) is larger than 13% (preferably larger than 15%) at a viewing angle of 0°, MTF@150lp/mm is larger than 22% (preferably larger than 25%) at a viewing angle of 115°, and MTF@100lp/mm is larger than 45% (preferably larger than 50%) at all viewing angles.

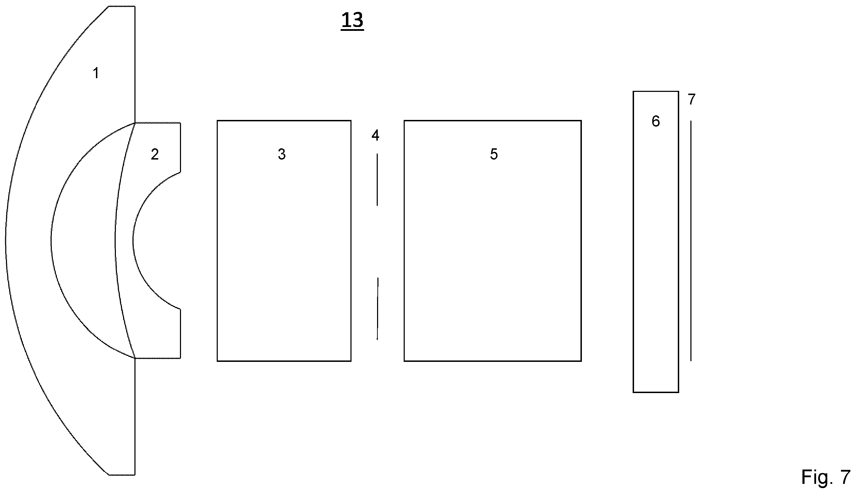

Fig. 7 shows a sensor system 13 which may be employed in a rigid tip portion 10 according to some embodiments of the invention. The sensor system 13 comprises the objective lens 14 and the image sensor with the sensor area 7. As shown in Fig. 7, the image sensor may be covered by a cover glass 6 which covers the sensor area 7. In this case, the cover glass 6 is located between the second condensing unit 5 and the sensor area 7.

A length through the objective lens 14 from an apex of the first meniscus lens 1 to the sensor area 7 may not be more than 10 mm. Thus, the sensor system 13 is particular suitable for a rigid tip portion 10 of an endoscope for inserting into a lumen of a human body such that even some processing circuit and/or control circuit may be arranged behind the sensor (seen from the object side). The sensor system 13 may be optimized to image a sphere located 8 mm in front of the apex of the first meniscus lens 1. In this case, the image circle should fit to the sensor area 7 of the sensor device. That is, the image circle is included in the sensor area 7. In an example, the image circle may have a diameter of not less than 2.08 mm and not more than 2.20 mm.

However, in some embodiments, the image circle includes the sensor area 7, or a major part of the sensor area 7 and the image circle overlap (e.g. more than 70%, more preferably more than 80%, or more preferably more than 90%). In some embodiments, if the diameter of the image circle is denoted di, the field of view of the objective lens 14 is denoted FoV, a diameter of the first meniscus lens 1 is denoted d1, and an F-number of the objective lens is denoted F, the following condition (8) may be fulfilled:

$$55 \le (di*FoV)/(d1*F) \quad (8).$$

In addition, the following condition (9) may be fulfilled:

$$(di*FoV)/(d1*F) \le 140 \quad (9).$$

In some embodiments, the lower limit according to condition (8) may be preferably 60 or 64. In some embodiments, the upper limit according to condition (9) may be preferably 135 or 131.

According to condition (8), the space is used particularly effective.

In the context of the present application, the term lens defines a transparent body having a front surface and a rear surface and a medium with an effective index larger than 1. The refractive index is (substantially) homogeneous throughout the lens. Typically, the lens surfaces are spherical. However, the lens surface may be aspherical, as long as it deviates from a spherical surface by not more than 10% (preferably 5%, more preferably 2%) of the respective radius. Typically, a material of the lenses is a glass. However, in some embodiments, one or more of the lenses may be made of plastic.

The front lens 1 (first meniscus lens) should preferably have a high resistance against acids and/or bases. The front lens should preferably be highly scratch resistant.

One or more of the lenses may have an antireflective coating or a coating to enhance resistance against acids and/or bases and/or to enhance scratch resistance. As long as this coating is not thicker than 10% (preferably 5%, more preferably 1%) of the maximum thickness of the lens, these coatings are included in the definition of the lens, although these coatings typically have a different refractive index than the remainder of the lens.

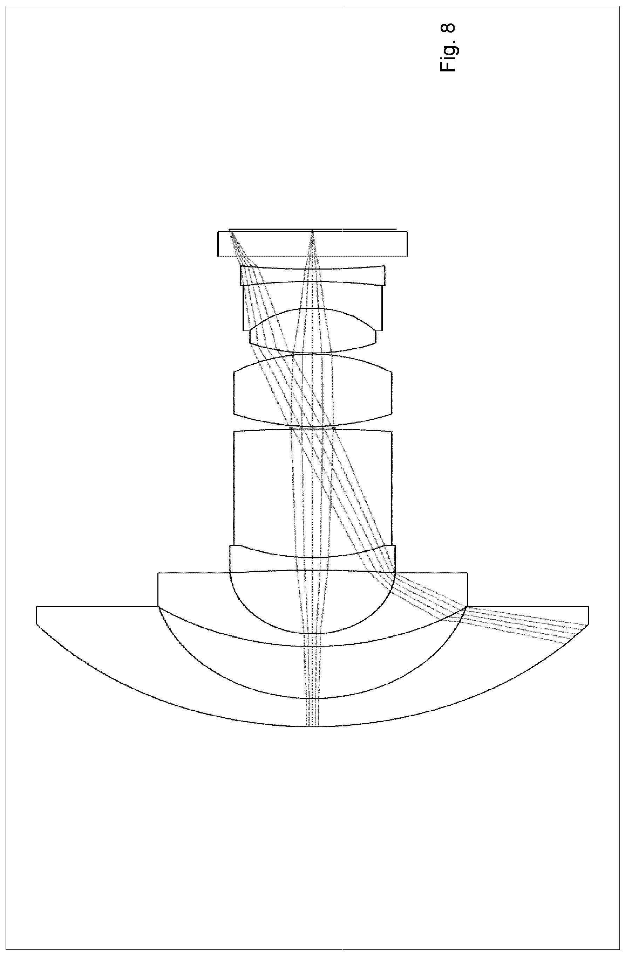

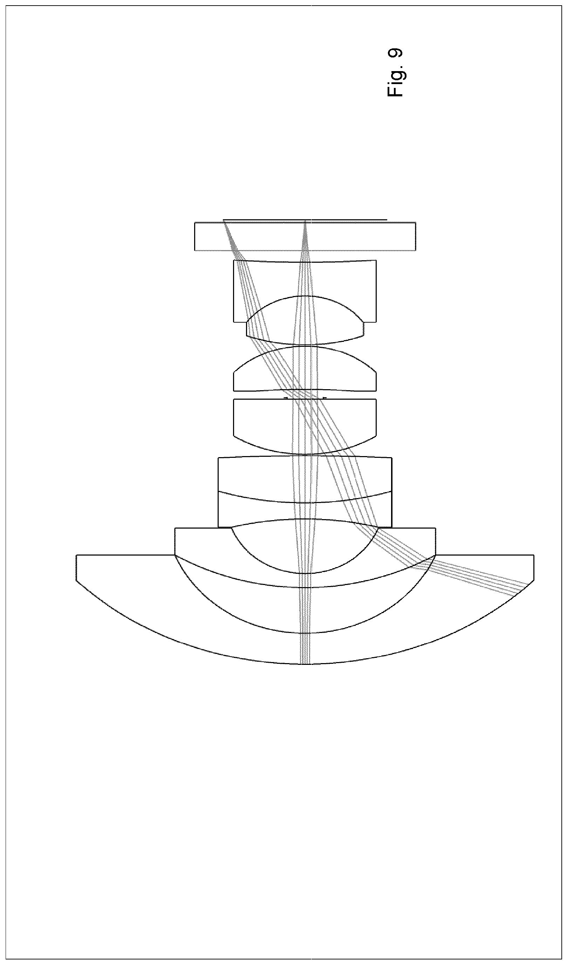

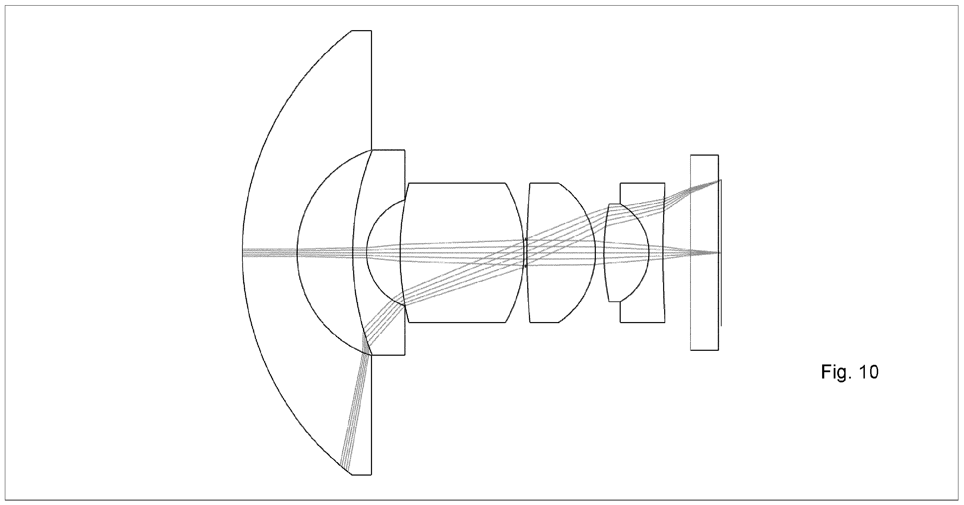

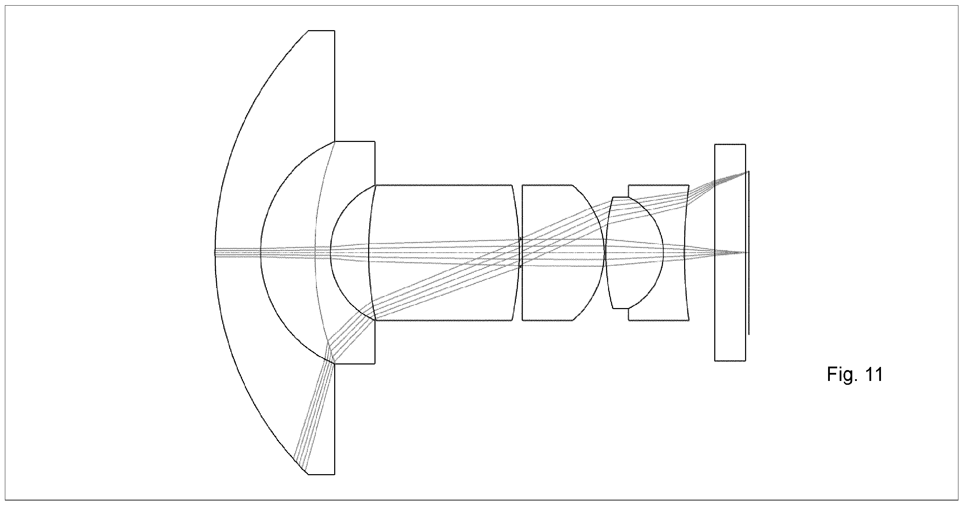

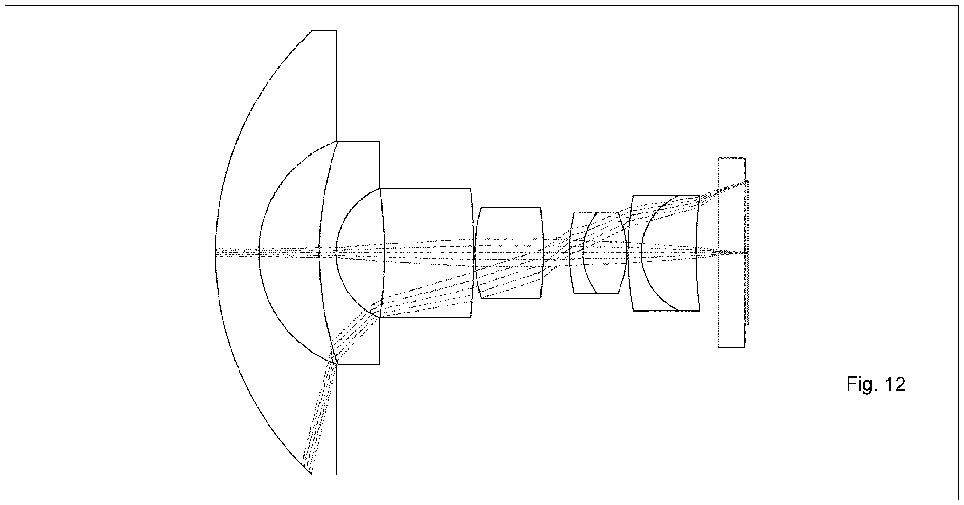

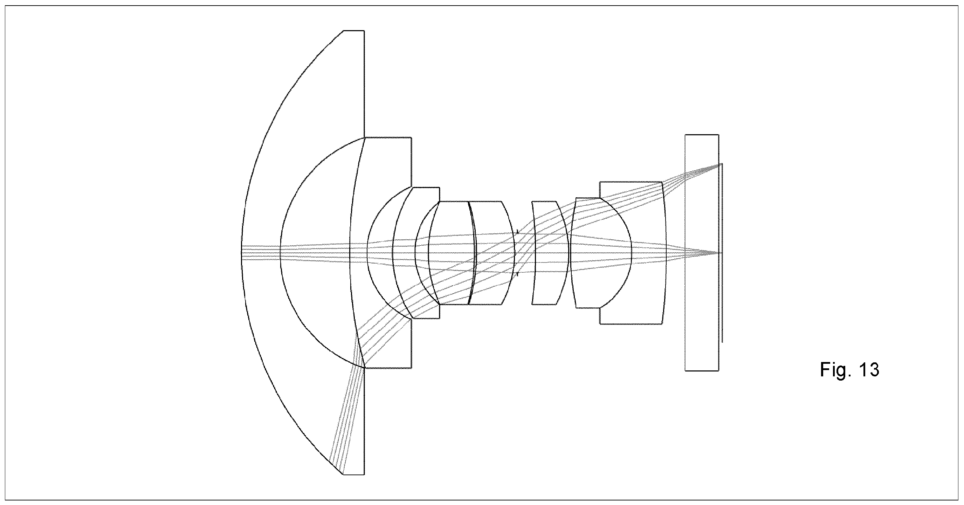

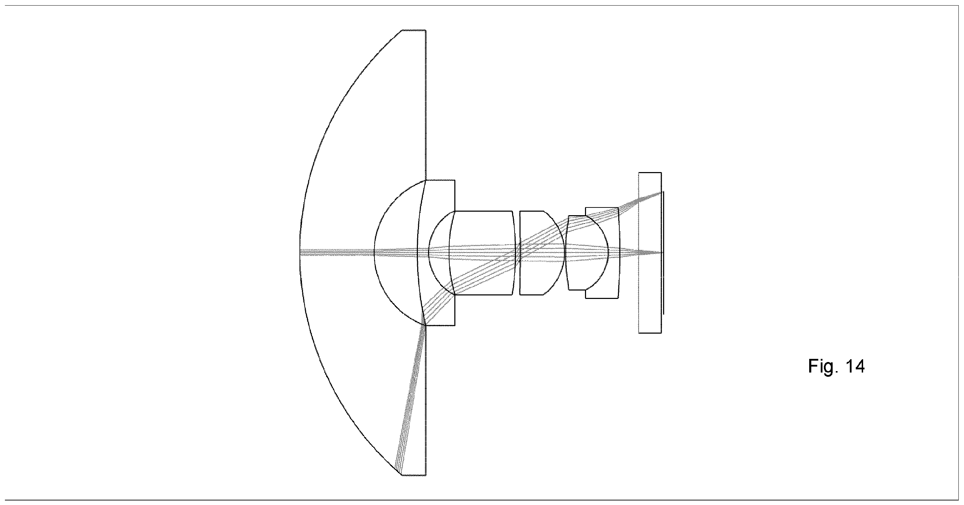

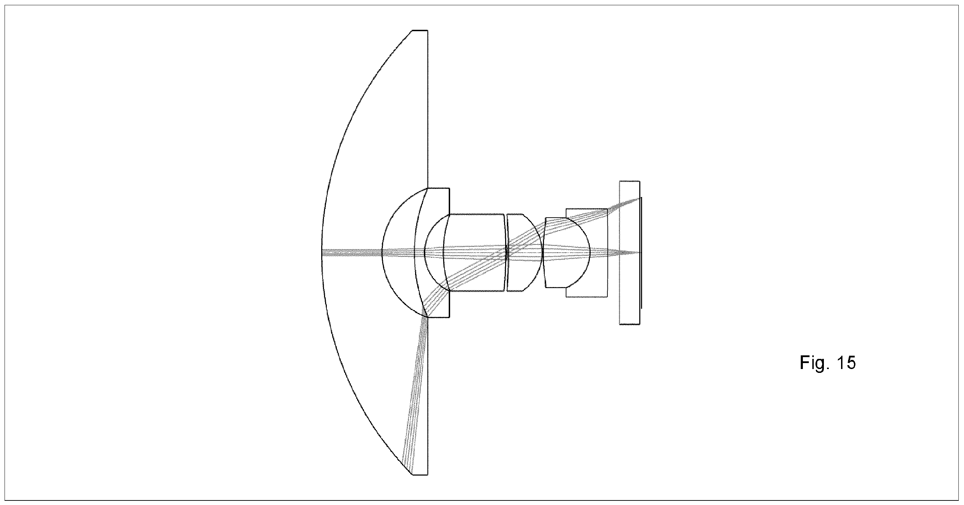

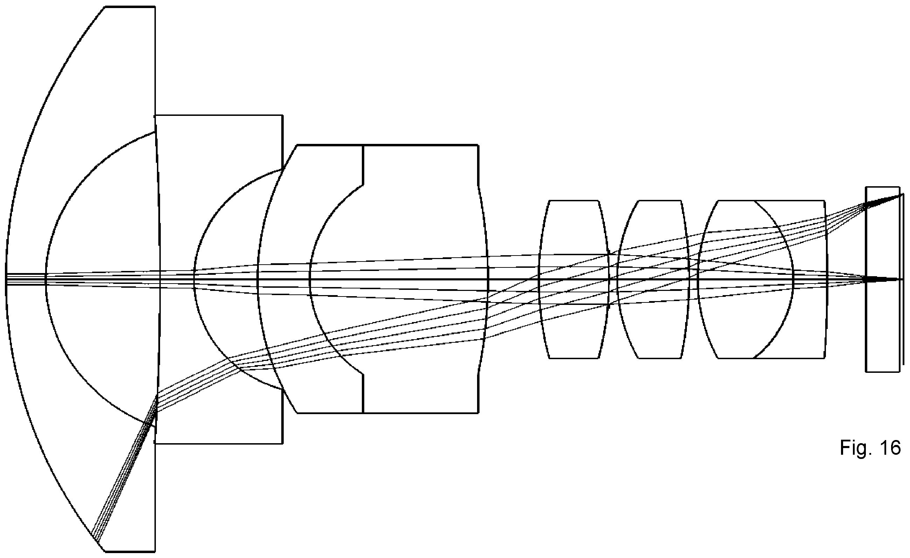

In Figures 8 to 15 and corresponding Tables 1 to 8, the objective lens 14 employed in rigid tip portions 10 according to embodiments 1 to 8 of the invention are specified at greater detail. In Figure 16 and corresponding Table 9, the objective lens 14 employed in rigid tip portions 10 according to comparative example 9 not covered by the claimed invention is specified at greater detail. Each of these figures shows a cross-sectional view through a sensor system 13 comprising the respective specified objective lens 14. In addition, a central light path and a light path at an extremal FoV are shown. The Tables indicate the respective lens parameters. Radiuses, thicknesses, and clear diameters are indicated in mm. Table 10 summarizes some of the relevant parameters for embodiments 1 to 8 and comparative example 9. Table 11 indicates the refractive indices and Abbe numbers of the glasses used for embodiments 1 to 8 and comparative example 9. However, the invention is not limited to these glasses.

TABLE 1: Lens data of Embodiment 1

| Surf | | Radius | Thickness | Glass | Clear Diam |
|---|---|---|---|---|---|
| OBJ | | 10 | 8 | | 0 |
| | 1 | 4,55868 | 0,45 | N-LASF31A | 6,997118 |
| | 2 | 2,025763 | 0,83 | | 3,897222 |
| | 3 | 3,325 | 0,2 | N-LASF31A | 3,924294 |
| | 4 | 1,05 | 1,019041 | | 2,095081 |
| | 5 | -14,08837 | 0,199991 | N-ZK7 | 2,092911 |
| | 6 | 2,237832 | 2,050761 | S-LAH88 | 1,824766 |
| | 7 | -12,34576 | 0,01991829 | | 2 |
| STO | | Infinity | 0,0197823 | | 0,508221 |
| | 9 | 2,659707 | 1,1507 | N-LAK34 | 2 |
| | 10 | -1.874717 | 0.01963665 | | 2 |
| | 11 | 2,117432 | 0,7167899 | S-LAH97 | 1,588087 |
| | 12 | -1,054733 | 0,4242363 | S-NPH3 | 1,591373 |
| | 13 | -5,81968 | 0,1999944 | S-NPH2 | 1,756799 |
| | 14 | 8,599747 | 0,196 | | 1,828844 |
| | 15 | Infinity | 0,4 | N-ZK7 | 2,4 |
| | 16 | Infinity | 0,04 | | 2,4 |
| IMA | | Infinity | | | 2,127379 |

TABLE 2: Lens data of Embodiment 2

| Surf | | Radius | Thickness | Glass | Clear Diam |
|---|---|---|---|---|---|
| OBJ | | 10 | 8 | | 0 |
| | 1 | 4,1 | 0,45 | N-LASF31A | 5,8 |
| | 2 | 1,78 | 0,65 | | 3,3 |
| | 3 | 3,164661 | 0,2 | N-LASF31A | 3,3 |
| | 4 | 0,985 | 0,78 | | 1,854 |
| | 5 | -3,73 | 0,23 | N-BAK4 | 1,854 |
| | 6 | 3,690252 | 0,67 | S-LAH79 | 2,2 |
| | 7 | -20,7 | 0,02008416 | | 2,2 |
| | 8 | 1,685 | 0,79 | S-LAH59 | 1,8 |
| | 9 | Infinity | 0,01991532 | | 1,2488 |
| STO | | Infinity | 0,1198657 | | 0,4533332 |
| | 11 | -7,76 | 0,6196602 | S-LAL19 | 1,2488 |
| | 12 | -1,26 | 0,01985869 | | 1,8 |
| | 13 | 2,175186 | 0,7 | S-LAH59 | 1,48 |

(continued)

| Surf | | Radius | Thickness | Glass | Clear Diam |
|---|---|---|---|---|---|
| | 14 | -0,91 | 0,48 | S-NPH3 | 1,48 |
| | 15 | 13,1 | 0,165 | | 1,8 |
| | 16 | Infinity | 0,4 | N-ZK7 | 2,8 |
| | 17 | Infinity | 0,04 | | 2,8 |
| IMA | | Infinity | | | 2,088328 |

TABLE 3: Lens data of Embodiment 3

| Surf | | Radius | Thickness | Glass | Clear Diam |
|---|---|---|---|---|---|
| OBJ | | 10 | 8 | | 0 |
| | 1 | 4 | 0,79 | N-LASF31A | 6,37094 |
| | 2 | 1,55 | 0,8 | | 2,945 |
| | 3 | 4 | 0,2 | S-LAH88 | 2,945 |
| | 4 | 0,8 | 0,48 | | 1,52 |
| | 5 | 4 | 1,78 | S-LAH55 | 2 |
| | 6 | -2 | 0,02 | | 2 |
| STO | | Infinity | 0,01975939 | | 0,3647792 |
| | 8 | 10,75 | 0,99 | S-LAL7 | 2 |
| | 9 | -1,2 | 0,125 | | 2 |
| | 10 | 3,5 | 0,6450499 | S-LAH59 | 1,4 |
| | 11 | -0,8 | 0,1998505 | S-NPH3 | 1,4 |
| | 12 | 16,42 | 0,396 | | 2 |
| | 13 | Infinity | 0,4 | N-ZK7 | 2,8 |
| | 14 | Infinity | 0,04 | | 2,8 |
| IMA | | Infinity | | | 2,102247 |

TABLE 4: Lens data of Embodiment 4

| Surf | | Radius | Thickness | Glass | Clear Diam |
|---|---|---|---|---|---|
| OBJ | | 10 | 8 | | 0 |
| | 1 | 4 | 0,59 | N-LASF31A | 5,738571 |
| | 2 | 1,55 | 0,705 | | 2,8675 |
| | 3 | 4 | 0,1998624 | S-LAH98 | 2,8675 |
| | 4 | 0,944 | 0,495 | | 1,74 |
| | 5 | 4 | 1,95 | S-LAH79 | 1,74 |
| | 6 | -4 | 0,02 | | 1,74 |
| STO | | Infinity | 0,02 | | 0,3347869 |
| | 8 | Infinity | 1,065 | N-LAK33B | 1,74 |
| | 9 | -1,122 | 0,02 | | 1,74 |
| | 10 | 2,925 | 0,745 | S-LAH59 | 1,44 |
| | 11 | -0,8 | 0,27 | S-NPH2 | 1,44 |
| | 12 | 6,0525 | 0,3938605 | | 1,74 |
| | 13 | Infinity | 0,4 | N-ZK7 | 2,8 |
| | 14 | Infinity | 0,04 | | 2,8 |
| IMA | | Infinity | | | 2,113744 |

TABLE 5: Lens data of Embodiment 5

| Surf | | Radius | Thickness | Glass | Clear Diam |
|---|---|---|---|---|---|
| OBJ | | 10 | 8 | | 0 |
| | 1 | 4,470595 | 0,64 | N-LASF31A | 6,559377 |
| | 2 | 1,75 | 0,8989142 | | 3,295538 |
| | 3 | 5,033335 | 0,25 | S-LAH98 | 3,295538 |
| | 4 | 1,0292 | 0,7127569 | | 1,908204 |
| | 5 | -7,05 | 1,318777 | N-SF5 | 1,904173 |
| | 6 | -10,46952 | 0,01993368 | | 1,904173 |
| | 7 | 2,44618 | 1,005609 | S-TIH57 | 1,340317 |
| | 8 | -5,766399 | 0,2006649 | | 1,340317 |
| STO | | Infinity | 0,1880398 | | 0,3875774 |
| | 10 | 2,461588 | 0,1998451 | N-SF66 | 1,2 |
| | 11 | 0,9250964 | 0,6519993 | S-FPM2 | 1,2 |
| | 12 | -1,436212 | 0,01971113 | | 1,2 |
| | 13 | 4,717489 | 0,1996736 | SF57HTULTRA | 1,7 |
| | 14 | 0,9249851 | 0,8086821 | N-LASF41 | 1,7 |
| | 15 | 6,792067 | 0,325 | | 1,66361 |
| | 16 | Infinity | 0,4 | N-ZK7 | 2,8 |
| | 17 | Infinity | 0,04 | | 2,8 |
| IMA | | Infinity | | | 2,133736 |

TABLE 6: Lens data of Embodiment 6

| Surf | | Radius | Thickness | Glass | Clear Diam |
|---|---|---|---|---|---|
| OBJ | | 10 | 8 | | 0 |
| | 1 | 3,457952 | 0,465 | N-LASF31A | 5,267703 |
| | 2 | 1,433196 | 0,8250966 | | 2,731529 |
| | 3 | 5,172053 | 0,205 | N-LASF31A | 2,731529 |
| | 4 | 0,851609 | 0,305 | | 1,575477 |
| | 5 | 1,352223 | 0,264521 | L-LAH91 | 1,550358 |
| | 6 | 0,7774331 | 0,16 | | 1,220027 |
| | 7 | 1,420964 | 0,5499949 | S-LAH93 | 1,220027 |
| | 8 | -2,37154 | 0,025 | | 1,220027 |
| | 9 | -1,973259 | 0,4522523 | N-LAK7 | 1,220027 |
| | 10 | -1,241067 | 0,03150913 | | 1,220027 |
| STO | | Infinity | 0,2157904 | | 0,4668453 |
| | 12 | -4,307316 | 0,3943424 | S-LAH65V | 1,220027 |
| | 13 | -1,300063 | 0,02 | | 1,220027 |
| | 14 | 3,031296 | 0,7344458 | N-LAK33B | 1,22755 |
| | 15 | 0,7485767 | 0,4092024 | S-NPH3 | 1,303808 |
| | 16 | -6,679741 | 0,223 | | 1,684501 |
| | 17 | Infinity | 0,4 | N-ZK7 | 2,8 |
| | 18 | Infinity | 0,04 | | 2,8 |
| IMA | | Infinity | | | 2,133043 |

TABLE 7: Lens data of Embodiment 7

| Surf | | Radius | Thickness | Glass | Clear Diam |
|---|---|---|---|---|---|
| OBJ | | 10 | 8 | | 0 |
| | 1 | 5,111192 | 1,3 | S-NBH55 | 7,762728 |

(continued)

| Surf | | Radius | Thickness | Glass | Clear Diam |
|---|---|---|---|---|---|
| | 2 | 1,337 | 0,7524318 | | 2,529821 |
| | 3 | 5,351464 | 0,2 | S-NBH55 | 2,529821 |
| | 4 | 0,8152746 | 0,3556025 | | 1,466356 |
| | 5 | 2,527006 | 1,164849 | N-SF66 | 1,460702 |
| | 6 | -4,35126 | 0,06336355 | | 1,460702 |
| STO | | Infinity | 0,01969143 | | 0,2854687 |
| | 8 | -36,85842 | 0,7725588 | S-LAH97 | 1,460702 |
| | 9 | -0,9044592 | 0,01950727 | | 1,460702 |
| | 10 | 3,323635 | 0,7508668 | S-LAH64 | 1,205833 |
| | 11 | -0,7232511 | 0,2031823 | S-NPH3 | 1,290018 |
| | 12 | -9,779682 | 0,324 | | 1,579365 |
| | 13 | Infinity | 0,4 | N-ZK7 | 2,8 |
| | 14 | Infinity | 0,04 | | 2,8 |
| IMA | | Infinity | | | 2,148502 |

TABLE 8: Lens data of Embodiment 8

| Surf | | Radius | Thickness | Glass | Clear Diam |
|---|---|---|---|---|---|
| OBJ | | 10 | 8 | | 0 |
| | 1 | 6,19235 | 1,182976 | N-BK7 | 8,692166 |
| | 2 | 1,337 | 0,633347 | | 2,52571 |
| | 3 | 3,182967 | 0,2 | LASF35 | 2,52571 |
| | 4 | 0,8152746 | 0,3666209 | | 1,494221 |
| | 5 | 2,237081 | 1,224584 | N-SF66 | 1,487515 |
| | 6 | -7,384379 | 0,02 | | 1,487515 |
| STO | | -7,384379 | 0,03342331 | | 0,2814958 |
| | 8 | -8,916423 | 0,6581249 | S-LAH65VS | 1,487515 |
| | 9 | -0,9127536 | 0,01998613 | | 1,487515 |
| | 10 | 3,656165 | 0,9232674 | N-LASF31A | 1,230488 |
| | 11 | -0,7223008 | 0,3520501 | S-NPH3 | 1,358825 |
| | 12 | -29,90218 | 0,22 | | 1,724946 |
| | 13 | Infinity | 0,4 | N-ZK7 | 2,8 |
| | 14 | Infinity | 0,04 | | 2,8 |
| IMA | | Infinity | | | 2,179126 |

TABLE 9: Lens data of comparative example 9

| Surf | Radius | Thickness | Glass | Clear Diam |
|---|---|---|---|---|
| OBJ | 10 | 8 | | 19,56464 |
| 1 | 5,7 | 0,47 | N-LASF31A | 6,918603 |
| 2 | 2 | 1,35 | | 3,74 |
| 3 | -35,027 | 0,4 | N-LASF41 | 4,18 |
| 4 | 1,46 | 0,75 | | 2,8 |
| 5 | 3,33 | 0,62 | N-SF66 | 3,4 |
| 6 | 1,46 | 2,1 | E-FD1 | 2,4 |
| 7 | -6,3 | 0,6 | | 2,4 |
| 8 | 4,0388 | 0,83 | S-FPL53 | 2 |

(continued)

| Surf | Radius | Thickness | Glass | Clear Diam |
|------|--------|-----------|-------|------------|
| 9 | -4,0388 | 0 | | 2 |
| STO | Infinity | 0,1 | | 0,6289341 |
| 11 | 2,1379 | 0,85 | S-FPL53 | 2 |
| 12 | -5,1195 | 0,1 | | 2 |
| 13 | 2,222 | 1,13 | S-FPL53 | 2 |
| 14 | -1,3 | 0,4 | FDS18 | 2 |
| 15 | -15,07 | 0,455 | | 2 |
| 16 | Infinity | 0,4 | N-BK7 | 2,151092 |
| 17 | Infinity | 0,045 | | 2,343473 |
| IMA | Infinity | | | 2,178735 |

TABLE 10: Miscellaneous data of Embodiments 1 to 8 and comparative example 9

| Emb. # | Figure | F-No. | f [mm] | TTL [mm] | FOV[°] | ø front lens/r4 | r2/r4 | r4/r3 | r2/r3 | r1/r4 |
|--------|--------|-------|--------|----------|--------|------------------|-------|-------|-------|-------|
| 1 | Fig. 8 | 2,8 | 0,54 | 7,937 | 235 | 6,66667 | 1,92952 | 0,31579 | 0,60932 | 4,34190 |
| 2 | Fig. 9 | 4 | 0,535 | 6,354 | 230 | 5,88832 | 1,80711 | 0,31122 | 0,56240 | 4,16244 |
| 3 | Fig. 10 | 4 | 0,526 | 6,886 | 230 | 7,96375 | 1,93750 | 0,20000 | 0,38750 | 5,00000 |
| 4 | Fig. 11 | 4 | 0,528 | 6,914 | 230 | 6,07945 | 1,64195 | 0,23600 | 0,38750 | 4,23729 |
| 5 | Fig. 12 | 4 | 0,517 | 8,228 | 235 | 6,08649 | 1,70068 | 0,20872 | 0,35497 | 4,17881 |
| 6 | Fig. 13 | 2,8 | 0,524 | 5,72 | 235 | 6,19036 | 1,68390 | 0,16454 | 0,27707 | 4,06345 |
| 7 | Fig. 14 | 4 | 0,5225 | 6,366 | 235 | 9,52165 | 1,63989 | 0,15236 | 0,24986 | 6,26886 |
| 8 | Fig. 15 | 4 | 0,5524 | 6,274 | 240 | 10,66111 | 1,63989 | 0,25614 | 0,42004 | 7,59475 |
| 9 | Fig. 16 | 4 | 0,6853 | 10,6 | 230 | 4,73972 | 1,36986 | -0,04168 | -0,05710 | 3,90411 |

TABLE 11

| Glass | Refractive index nd | Abbe number vd |
|-------|---------------------|----------------|
| N-LASF31A | 1,883 | 40,76 |
| S-NBH55 | 1,8 | 29,84 |
| N-BK7 | 1,5168 | 64,17 |
| LASF35 | 2,02204 | 29,06 |
| S-LAH98 | 1,95375 | 32,32 |
| S-LAH88 | 1,9165 | 31,6 |
| L-LAH91 | 1,7645 | 49,09 |
| S-LAH93 | 1,90525 | 35,04 |
| S-LAH55 | 1,83481 | 42,7 |
| N-SF5 | 1,67271 | 32,25 |

(continued)

| Glass | Refractive index nd | Abbe number vd |
|---|---|---|
| N-LAK7 | 1,6516 | 58,52 |
| N-LAK33B | 1,755 | 52,3 |
| N-LAK34 | 1,72916 | 54,5 |
| N-BAK4 | 1,56883 | 55,98 |
| S-TIH57 | 1,963 | 24,11 |
| N-SF66 | 1,92286 | 20,88 |
| S-FPM2 | 1,59522 | 67,74 |
| S-LAH79 | 2,0033 | 28,3 |
| S-LAH59 | 1,816 | 46,6 |
| S-LAH64 | 1,788 | 47,4 |
| S-LAH65V | 1,804 | 46,6 |
| S-LAH65VS | 1,804 | 46,53 |
| S-LAH97 | 1,755 | 52,32 |
| S-LAL19 | 1,72916 | 54,09 |
| S-LAL7 | 1,6516 | 58,55 |
| N-SF57HULTRA | 1,84666 | 23,78 |
| N-LASF41 | 1,83501 | 43,13 |
| S-NPH3 | 1,95906 | 17,47 |
| S-NPH2 | 1,92286 | 18,9 |
| N-ZK7 | 1,50847 | 61,19 |
| E-FD1 | 1,71736 | 29,5 |
| S-FPL53 | 1,43875 | 94,93 |
| FDS18 | 1,9459 | 17,98 |

**Claims**

1. A rigid tip portion (10) for an endoscope for inserting into a lumen of a human body, wherein

the rigid tip portion has a center axis extending from a distal end of the rigid tip portion to a proximal end of the rigid tip portion;
at each position along the center axis, a respective cross-section of the rigid tip portion perpendicular to the center axis is elliptical with a long axis and a short axis not longer than the long axis;
the long axis and the short axis cross on the center axis;
the rigid tip portion accommodates a sensor system comprising
a single objective lens (14); and
an image sensor (7), wherein
a sensor area of the image sensor for photoelectrically converting an optical image taken through the single objective lens into an electrical image signal is arranged in an image plane of the single objective lens;
a front lens (1) on an object side of the single objective lens is arranged at the distal end of the rigid tip portion, **characterized in that**
the single objective lens has a field of view of not less than 180°;
the image plane is located such that the objective lens images a portion of a sphere on the image plane; the sphere has a radius of not less than 8 mm and not more than 12 mm; a center of the sphere is located not less than 0 mm and not more than 4 mm towards the image sensor from an apex of the objective lens on a side opposite to a side where the image sensor is located; and
the objective lens consists of, in order from the object side to an image side:

the front lens being a first meniscus lens having a convex side towards the object side;
a second lens (2);
a first condensing unit (3) configured to condense light from the second lens;
an aperture stop (4);
a second condensing unit (5) configured to condense light from the aperture stop; wherein

a total number of lenses in the first and second condensing units is not less than 4 and not more than 6;
the second lens is a second meniscus lens (2) having a convex side towards the object side;
an apex of the second meniscus lens is spaced apart from the first meniscus lens;
if a radius of the first meniscus lens on the image side is denoted r2, a radius of the second meniscus lens on the object side is denoted r3, and a radius of the second meniscus lens on the image side is denoted r4, at least two of the following three conditions are fulfilled:

$$r2/r4 \geq 1.40;$$

$$r4/r3 \geq 0.111;$$

and

$$r2/r3 \geq 0.16.$$

2. The rigid tip portion according to claim 1, wherein, at each position along the center axis, the respective cross-section is circular such that the respective long axis and the respective short axis have a respective same length.

3. The rigid tip portion according to any of claims 1 and 2, wherein
a maximum length of the long axes for all the cross-sections is not more than 18 mm.

4. The rigid tip portion according to any of claims 1 to 3, wherein a length of the rigid tip portion along the center axis is not larger than 30 mm.

5. The rigid tip portion according to any of claims 1 to 4, wherein the image plane is arranged perpendicular to the center axis or inclined to the center axis at an angle of not less than 75°.

6. The rigid tip portion according to any of claims 1 to 4, wherein at least one of

a thickness of the front lens at a center of the front lens is not less than 0.45 mm; and
a thickness of the front lens at an edge of the front lens is not less than 0.3 mm.

7. The rigid tip portion according to any of claims 1 to 6, wherein at least one of the following conditions is fulfilled:

$$1.98 \geq r2/r4;$$

$$0.35 \geq r4/r3;$$

and

$$0.65 \geq r2/r3.$$

8. The rigid tip portion according to any of claims 1 to 7, wherein at least one of the following conditions is fulfilled:

the first condensing unit consists of one lens or two lenses or three lenses; and
the second condensing unit consists of three lenses or four lenses.

9. The rigid tip portion according to any of claims 1 to 8, wherein,
if a radius of the first meniscus lens on the object side is denoted r1, the following condition is fulfilled:

$$4.0 \leq r1/r4 \leq 7.6.$$

10. The rigid tip portion according to any of claims 1 to 9, wherein a diameter of the first meniscus lens is not larger than 7 mm.

11. The rigid tip portion according to any of claims 1 to 10, wherein an optical length from an apex of the first meniscus lens to the sensor area is not more than 10 mm.

12. The rigid tip portion according to claim 11, wherein

an image circle of a sphere located 8 mm in front of the apex of the first meniscus lens on the sensor area is included in the sensor area, and
a chief ray angle at each location of the image circle is not larger than 30°.

13. An endoscope, comprising

a flexible tube (17);
an angulation segment (12); and
the rigid tip portion according to any of claims 1 to 12, wherein
a proximal end of the angulation segment is attached to a distal end of the flexible tube;
the proximal end of the rigid tip portion is attached to a distal end of the angulation segment, and
the endoscope is for inserting into the lumen of the human body.

**Patentansprüche**

1. Ein starrer Spitzenanteil (10) für ein Endoskop zum Einführen in eine Öffnung eines menschlichen Körpers, wobei

der starre Spitzenanteil eine Zentralachse aufweist, die sich von einem distalen Ende des starren Spitzenanteils zu einem proximalen Ende des starren Spitzenanteils erstreckt;
an jeder Position entlang der Zentralachse ein jeweiliger Querschnitt des starren Spitzenanteils senkrecht zu der Zentralachse elliptisch mit einer langen Achse und einer kurzen Achse, die nicht länger als die lange Achse ist, ist;
die lange Achse und die kurze Achse sich an der Zentralachse kreuzen;
der starre Spitzenanteil ein Sensorsystem beherbergt, das aufweist:

eine einzelne Objektivlinse (14); und
einen Bildsensor (7), wobei
eine Sensorfläche des Bildsensors zum fotoelektrischen Umwandeln eines optischen Bildes, das durch die einzelne Objektivlinse aufgenommen wird, in ein elektrisches Bildsignal in einer Bildebene der einzelnen Objektivlinse angebracht ist;
eine vordere Linse (1) auf einer Objektseite der einzelnen Objektivlinse an dem distalen Ende des starren Spitzenanteils angebracht ist,
**dadurch gekennzeichnet, dass**
die einzelne Objektivlinse ein Blickfeld von nicht weniger als 180° hat;
die Bildebene so lokalisiert ist, dass die Objektivlinse einen Anteil einer Kugel auf der Bildebene abbildet, wobei die Kugel einen Radius von nicht weniger als 8 mm nicht mehr als 12 mm hat; ein Zentrum der Kugel nicht weniger als 0 mm und nicht mehr als 4 mm hin zu dem Bildsensor von einem Scheitelpunkt der Objektivlinse auf einer Seite gegenüber einer Seite, an der der Bildsensor lokalisiert ist, lokalisiert ist; und
die Objektivlinse in einer Reihenfolge von der Objektseite zu einer Bildseite besteht aus:

der vorderen Linse, die eine erste Meniskuslinse mit einer konvexen Seite hin zu der Objektseite ist;
einer zweiten Linse (2);
einer ersten Kondensiereinheit (3), die konfiguriert ist, Licht von der zweiten Linse zu kondensieren;
einer Blende (4);
einer zweiten Kondensiereinheit (5), die konfiguriert ist, Licht von der Blende zu kondensieren; wobei
eine Gesamtzahl an Linsen in den ersten und zweiten Kondensiereinheiten nicht kleiner als 4 und nicht größer als 6 ist;
die zweite Linse eine zweite Meniskuslinse (2) mit einer konvexen Seite hin zu der Objektseite ist;
ein Scheitelpunkt der zweiten Meniskuslinse von der ersten Meniskuslinse beabstandet ist;

wenn ein Radius der ersten Meniskuslinse auf der Bildseite als r2 bezeichnet wird, ein Radius der zweiten Meniskuslinse auf der Objektseite als r3 bezeichnet wird, und ein Radius der zweiten Meniskuslinse auf der Bildseite als r4 bezeichnet wird, zumindest zwei der folgenden drei Bedingungen erfüllt sind:

$$r2/r4 \geq 1{,}40;$$

$$r4/r3 \geq 0{,}111;$$

und

$$r2/r3 \geq 0{,}16.$$

2. Starrer Spitzenanteil nach Anspruch 1, wobei an jeder Position entlang der Zentralachse der jeweilige Querschnitt kreisförmig ist, sodass die jeweilige lange Achse und die jeweilige kurze Achse eine jeweilige gleiche Länge haben.

3. Starrer Spitzenanteil nach einem der Ansprüche 1 und 2, wobei eine maximale Länge der langen Achsen für alle Querschnitte nicht größer als 18 mm ist.

4. Starrer Spitzenanteil nach einem der Ansprüche 1 bis 3, wobei eine Länge des starren Spitzenanteils entlang der Zentralachse nicht größer als 30 mm ist.

5. Starrer Spitzenanteil nach einem der Ansprüche 1 bis 4, wobei die Bildebene senkrecht zu der Zentralachse oder geneigt zu der Zentralachse mit einem Winkel von nicht weniger als 75° angebracht ist.

6. Starrer Spitzenanteil nach einem der Ansprüche 1 bis 4, wobei zumindest eine Dicke der vorderen Linse an einem Zentrum der vorderen Linse nicht weniger als 0,45 mm ist; oder eine Dicke der vorderen Linse an einer Kante der vorderen Linse nicht weniger als 0,3 mm ist.

7. Starrer Spitzenanteil nach einem der Ansprüche 1 bis 6, wobei zumindest eine der folgenden Bedingungen erfüllt ist:

$$1{,}98 \geq r2/r4;$$

$$0{,}35 \geq r4/r3;$$

and

$$0{,}65 \geq r2/r3.$$

8. Starrer Spitzenanteil nach einem der Ansprüche 1 bis 7, wobei zumindest eine der folgenden Bedingungen erfüllt ist:

die erste Kondensiereinheit besteht aus einer Linse oder zwei Linsen oder drei Linsen; und
die zweite Kondensiereinheit besteht aus drei Linsen oder vier Linsen.

9. Starrer Spitzenanteil nach einem der Ansprüche 1 bis 8, wobei, wenn ein Radius der ersten Meniskuslinse auf der Objektseite als r1 bezeichnet wird, die folgende Bedingung erfüllt ist:

$$4{,}0 \leq r1/r4 \leq 7{,}6.$$

10. Starrer Spitzenanteile nach einem der Ansprüche 1 bis 9, wobei ein Durchmesser der ersten Meniskuslinse nicht größer als 7 mm ist.

**EP 3 767 364 B1**

**11.** Starrer Spitzenanteil nach einem der Ansprüche 1 bis 10, wobei eine optische Länge von einem Scheitelpunkt der ersten Meniskuslinse zu der Sensorfläche nicht größer als 10 mm ist.

**12.** Starrer Spitzenanteil nach Anspruch 11, wobei

ein Bildkreis einer Kugel, die 8 mm vor dem Scheitelpunkt der ersten Meniskuslinse lokalisiert ist, auf der Sensorfläche in der Sensorfläche enthalten ist, und
ein Hauptstrahlwinkel an jedem Ort des Bildkreises nicht größer als 30° ist.

**13.** Endoskop, das aufweist:

eine flexible Röhre (17);
ein Angulationssegment (12); und
den starren Spitzenanteil nach einem der Ansprüche 1 bis 12, wobei
ein proximales Ende des Angulationssegments an einem distalen Ende der flexiblen Röhre angebracht ist;
das proximale Ende des starren Spitzenanteils an einem distalen Ende des Angulationssegments angebracht ist, und
das Endoskop zum Einführen in die Öffnung des menschlichen Körpers geeignet ist.

**Revendications**

**1.** Partie de pointe rigide (10) pour un endoscope à insérer dans une lumière du corps humain, dans laquelle

la partie de pointe rigide a un axe central qui s'étend d'une extrémité distale de la partie de pointe rigide à une extrémité proximale de la partie de pointe rigide ;
à chaque position le long de l'axe central, une section transversale respective de la partie de pointe rigide perpendiculaire à l'axe central est elliptique avec un axe long et un axe court qui n'est pas plus long que l'axe long ;
l'axe long et l'axe court se croisent sur l'axe central ;
la partie de pointe rigide reçoit un système de capteur comprenant
une lentille d'objectif unique (14) ; et
un capteur d'image (7), dans lequel système de capteur
une zone de capteur du capteur d'image pour la conversion photoélectrique d'une image optique prise à travers l'objectif unique en un signal d'image électrique est disposée dans un plan d'image de la lentille d'objectif unique ;
une lentille frontale (1) du côté de l'objet de lentille d'objectif unique est disposée à l'extrémité distale de la partie de pointe rigide,
**caractérisé en ce que**
la lentille d'objectif unique a un champ de vision de pas moins de 180° ;
le plan d'image est situé de telle sorte que la lentille d'objectif image une partie d'une sphère sur le plan d'image ;
la sphère a un rayon de pas moins de 8 mm et de pas plus de 12 mm ; un centre de la sphère est situé à pas moins de 0 mm et à de pas plus de 4 mm vers le capteur d'image à partir d'un sommet de la lentille d'objectif sur un côté opposé à un côté où se trouve le capteur d'image ; et
dans l'ordre du côté de l'objet au côté de l'image, la lentille d'objectif se compose des éléments suivants :

la lentille frontale est une première lentille ménisque ayant un côté convexe vers le côté de l'objet ;
une deuxième lentille (2) ;
une première unité de condensation (3) configurée pour condenser la lumière provenant de la deuxième lentille ;
une butée d'ouverture (4) ;
une deuxième unité de condensation (5) configurée pour condenser la lumière provenant de la butée d'ouverture ; dans laquelle lentille d'objectif
un nombre total de lentilles dans la première et la deuxième unité de condensation n'est pas inférieur à 4 et n'est pas supérieur à 6 ;
la deuxième lentille est une deuxième lentille ménisque (2) ayant un côté convexe vers le côté de l'objet ;
un sommet de la deuxième lentille ménisque est espacé de la première lentille ménisque ;
si un rayon de la première lentille ménisque du côté de l'image est noté r2, un rayon de la deuxième lentille ménisque du côté de l'objet est noté r3, et un rayon de la deuxième lentille ménisque du côté de l'image est noté r4, au moins deux des trois conditions suivantes sont remplies :

$$r2/r4 \geq 1,40 \; ;$$

$$r4/r3 \geq 0,111 \; ;$$

et

$$r2/r3 \geq 0,16.$$

**2.** Partie de pointe rigide selon la revendication 1, dans laquelle, à chaque position le long de l'axe central, la section transversale respective est circulaire de sorte que l'axe long respectif et l'axe court respectif ont la même longueur.

**3.** Partie de pointe rigide selon l'une des revendications 1 et 2, dans laquelle une longueur maximale des axes longs de toutes les sections transversales n'est pas supérieure à 18 mm.

**4.** Partie de pointe rigide selon l'une des revendications 1 à 3, dans laquelle une longueur de la partie de pointe rigide le long de l'axe central n'est pas supérieure à 30 mm.

**5.** Partie de pointe rigide selon l'une des revendications 1 à 4, dans laquelle le plan d'image est disposé perpendiculairement à l'axe central ou incliné par rapport à l'axe central à un angle de pas moins de 75°.

**6.** Partie de pointe rigide selon l'une des revendications 1 à 4, dans laquelle au moins

une épaisseur de la lentille frontale au centre de la lentille frontale n'est pas inférieure à 0,45 mm ; et
une épaisseur de la lentille frontale sur un bord de la lentille frontale n'est pas inférieure à 0,3 mm.

**7.** Partie de pointe rigide selon l'une des revendications 1 à 6, dans laquelle au moins l'une des conditions suivantes est remplie :

$$1,98 \geq r2/r4 \; ;$$

$$0,35 \geq r4/r3 \; ;$$

et

$$0,65 \geq r2/r3.$$

**8.** Partie de pointe rigide selon l'une des revendications 1 à 7, dans laquelle au moins l'une des conditions suivantes est remplie :

la première unité de condensation est constituée d'une lentille, de deux lentilles ou de trois lentilles ; et
la deuxième unité de condensation est constituée de trois lentilles ou de quatre lentilles.

**9.** Partie de pointe rigide selon l'une des revendications 1 à 8, dans laquelle, si le rayon de la première lentille ménisque du côté de l'objet est noté r1, la condition suivante est remplie :

$$4,0 \leq r1/r4 \leq 7,6.$$

**10.** Partie de pointe rigide selon l'une des revendications 1 à 9, dans laquelle un diamètre de la première lentille ménisque n'est pas supérieure à 7 mm.

**11.** Partie de pointe rigide selon l'une des revendications 1 à 10, dans laquelle une longueur optique entre un sommet de la première lentille ménisque et la zone de capteur ne dépasse pas 10 mm.

**12.** Partie de pointe rigide selon la revendication 11, dans laquelle

un cercle d'image d'une sphère située à 8 mm en avant du sommet de la première lentille ménisque sur la zone de capteur est inclus dans la zone de capteur, et
un angle de faisceau principal à chaque emplacement du cercle d'image n'est pas supérieur à 30°.

**13.** Endoscope comprenant

un tube flexible (17) ;
un segment d'angulation (12) ; et
la partie de pointe rigide selon l'une des revendications 1 à 12, dans laquelle,
une extrémité proximale du segment d'angulation est attaché à une extrémité distale du tube flexible ;
l'extrémité proximale de la partie de pointe rigide est attaché à l'extrémité distale du segment d'angulation, et
l'endoscope est destiné à être inséré dans la lumière du corps humain.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1629764 A1
- JP H10309259 A
- WO 2017027749 A1
- US 20070213590 A1
- US 20170146778 A1
- JP 2008058387 A
- WO 2009150653 A1